(19) Europäisches Patentamt

European Patent Office

Office européen des brevets

(11) Numéro de publication: **0 481 148 A1**

## (12) DEMANDE DE BREVET EUROPEEN

(21) Numéro de dépôt: **90402919.6**

(22) Date de dépôt: **17.10.90**

(51) Int. Cl.5: **A61K 37/22**, A61K 31/20,
//(A61K37/22,31:20,31:07),
(A61K31/20,31:07)

(43) Date de publication de la demande:
**22.04.92 Bulletin 92/17**

(84) Etats contractants désignés:
**AT BE CH DE DK ES FR GB GR IT LI LU NL SE**

(71) Demandeur: DIETLIN, François
**17, rue du Maréchal Foch**
**F-78110 Le Vésinet(FR)**
Demandeur: Fredj, Danièle
**13bis Chemin des Rougemonts**
**F-91190 Gif S/Yvette(FR)**

(72) Inventeur: DIETLIN, François
**17, rue du Maréchal Foch**
**F-78110 Le Vésinet(FR)**
Inventeur: Fredj, Danièle
**13bis Chemin des Rougemonts**
**F-91190 Gif S/Yvette(FR)**

(74) Mandataire: Burtin, Jean-François
**Cabinet GEFIB, 59, rue Edouard-Vaillant**
**F-92300 Levallois-Perret(FR)**

(54) **Nouvelles compositions pharmaceutiques, leur procédé d'obtention et leur emploi en thérapeutique tumorale.**

(57) L'invention se rapporte au domaine de la chimie pharmaceutique et notamment au domaine de la thérapeutique tumorale.

Elle a pour objet de nouvelles compositions pharmaceutiques caractérisées en ce qu'elles renferment à titre de principes actifs une huile hyper-oxygénée et un dérivé oxygéné du rétinène en association avec un véhicule ou un excipient inerte non irritant pharmaceutiquement compatible.

L'invention concerne également un procédé d'obtention desdites formes pharmaceutiques.

La présente invention se rapporte au domaine de la chimie pharmaceutique et notamment au domaine de la thérapeutique humaine.

Elle a plus particulièrement pour objet de nouvelles compositions pharmaceutiques destinées principalement à la thérapeutique tumorale et à leur procédé d'obtention.

Elle a spécifiquement pour objet de nouvelles compositions pharmaceutiques caractérisées en ce qu'elles renferment à titre de principes actifs une huile hyper-oxygénée et un dérivé oxygéné du rétinène en association ou en mélange avec un véhicule ou un excipient inerte non irritant pharmaceutiquement compatible.

On entend sous la dénomination huile hyper-oxygénée, un corps gras liquide à température ordinaire d'origine animale ou végétale, présentant une ou plusieurs fonctions hydropéroxydes et résultant de l'insufflation d'air ou d'oxygène pur ou dilué dans une matière grasse liquide à température ordinaire et possédant au moins deux double liaisons éthyléniques.

L'huile hyper-oxygénée peut dériver d'une huile poly-insaturée d'origine végétale non siccative comme l'huile d'amandes douces, l'huile d'olives, l'huile de Bourrache, l'huile de pépins de cassis, l'huile de pépins de raisins, l'huile d'Onagre (Oenothera Lamarckiana), l'huile de Colza, l'huile d'oeillette ou d'huile de Maïs ou bien d'une huile poly-insaturée d'origine animale comme les huiles de poisson comme l'huile de foie de thon, l'huile de foie de morue ou l'huile de foie de fletan. Elles sont obtenues par barbottage prolongé d'air ou d'oxygène dilué dans ces huiles, éventuellement sous irradiation ultraviolette ainsi qu'il est décrit dans la demande de brevet européen 117.962.

L'huile hyper-oxygénée présentement préférée, est celle obtenue par peroxygénation de l'huile de Maïs ou l'huile d'arachide.

Le dérivé oxygéné du rétinène est un composé du type pentaénique en $C_{20}$ présentant en position 1, un hydroxyméthyle (rétinol) libre ou estérifié, un formyle (rétinal) ou bien un hydroxy carbonyle (acide rétinoïque) libre ou estérifié.

Les composés peuvent présenter une configuration cis ou trans, intégrale ou mélangée. On pourra de ce fait, inclure sous cette dénomination :

- l'acide rétinoique de configuration CIS
- la Trétinoine ou acide rétinoique de configuration entièrement trans
- l'Etretinate ou retinoate d'éthyle entièrement trans
- l'Isotretinoine
- l'acitrétine

Parmi les esters de rétinol, on pourra citer plus particulièrement les esters d'acide aliphatique comme l'acétate ou le propionate, les esters d'acides hydroxylés comme l'ascorbate de rétinol ou les esters d'acides aliphatiques à longue chaine comme le palmitate ou le stéarate de rétinol.

Ces compositions pharmaceutiques selon l'invention sont destinées à l'usage externe et se présentent de préférence sous forme solide, ou liquide, comme par exemple des poudres, des pommades, des crèmes, des lotions, des laits, des gels, des émulsions huile dans l'eau éventuellement encapsulées dans des microglobules ou dans des liposomes plurilamellaires.

Le rapport pondéral entre ces deux principes actifs peut varier dans de larges proportions et notamment de 10 parties d'huile hyper oxygénée pour une partie de dérivé du Rétinène à 1 partie d'huile hyper-oxygénée pour 5 parties de dérivé oxygéné du Rétinène.

Les critères de choix des proportions respectives de principes actifs résident surtout dans l'ancienneté des lésions à traiter et peuvent requérir dans ces cas, une proportion plus grande de dérivé du Rétinène.

Les huiles hyper-oxygénées jouent un rôle significatif dans les compositions selon l'invention car elles paraissent favoriser le passage du dérivé du rétinène dans le derme et sa fixation sur les lésions à traiter.

Les compositions pharmaceutiques selon l'invention contribuent au traitement des lésions cutanées liées au syndrôme de Kaposi. Celles-ci se caractérisent par des taches plus ou moins étendues de dépigmentation, des zones oedematiées par suite d'infiltration de liquides dans le derme et surtout par la survenue de nodosités ou de protuberances plus ou moins dures et plus ou moins volumineuses. Ces lésions affectent plus particulièrement le visage, les mains, le cou et le dos.

Le syndrôme de Kaposi est un cancer à manifestation cutanée qui se produit spontanément et, en général, d'une manière initiale dès la survenue parallèle d'autres tumeurs. En particulier, le syndrôme de Kaposi est une des formes d'évolution du syndrôme immuno-dépresseur acquis. Les malades atteints du SIDA présentent dans une phase avancée de cette maladie des nodosités pigmentées dont le volume et la coloration se renforcent au cours de l'évolution de cette maladie.

Jusqu'ici, on ne connaissait pas de médicament apte à traiter cette maladie et les essais déjà menés avec l'acide rétinoïque seul n'ont pas fourni de résultats. L'acide rétinoïque permet de décaper la peau, ou l'épiderme mais son action est seulement décapante et nullement curative. Ce traitement purement correctif, n'empêche pas le développement ou le durcissement des modules. De ce fait, le seul traitement pratiqué jusqu'ici est une excision.

L'huile hyper-oxygénée a déjà trouvé un emploi en thérapeutique, notamment en thérapeutique broncho-pulmonaire mais son instabilité ne permettait d'en faire un médicament, en solution ou suspension permanente.

Dans les présentes compositions, objet de l'invention, l'huile hyper-oxygénée favorise le passage des dérivés du rétinène et leur confère une efficacité certaine.

Les quantités administrées peuvent varier dans de larges proportions de manière à pouvoir moduler d'une façon importante le schéma thérapeutique en fonction notamment de l'ancienneté des lésions.

D'une manière préférée, on utilise des lotions, des crèmes ou des sprays de préparations gazeuses, fluides ou sèches, renfermant de O,5 à 10 % de principes actifs. On préfère à cet égard des compositions pharmaceutiques renfermant de 1 à 2 % du dérivé oxygéné du rétinène et de O,5 à 5 % d'huile hyperoxygénée.

Une composition pharmaceutique tout à fait préférée actuellement est celle qui contient 1 % d'acide rétinoïque ou de Tretinoine et de 2 à 5% d'huile hyperoxygénée dans un excipient gélifiant.

L'efficacité des compositions pharmaceutiques selon l'invention est appréciée par la mesure de la taille des lésions, de leur pigmentation, de l'importance des phénomènes d'oedème et de la consistance des nodosités.

On constate que quelle que soit l'ancienneté ou l'importance des lésions, la coloration des lésions, il se produit un changement complet dans la consistance des nodules. Dans la mesure où dans le traitement éventuel du syndrôme de Kaposi, la taille, le volume et la coloration des nodules peuvent évoluer dans de larges proportions, il est souhaitable de disposer d'une médicament actif sur toutes les manifestations et efficace rapidement sur les lésions cutanées.

Pour la réalisation des compositions pharmaceutiques selon l'invention, on utilise les techniques habituelles du savoir faire industriel de la pharmacotechnie comme par exemple une mise en solution des principes actifs dans un excipient lipidique inerte, addition d'un agent émulsionnant comme le stéarate de polyéthylène glycol additionné ou non d'agents de stabilisation, de conservation ou de dispersion. Cette solution est ensuite diluée -sous agitation- par un milieu aqueux renfermant en outre des produits d'aromatisation ou de coloration, ou un agent épaississant. Il se forme ainsi un gel ou une crème ou une lotion selon le degré de viscosité. Ces préparations sont réparties en tubes, en pots ou de préférence en sachets plastique mono-doses.

Comme agent épaississant, on peut utiliser une alcoylcellulose comme la méthylcellulose, l'éthylcellulose, l'hydroxypropylcellulose ou l'hydroxypropyl méthylcellulose ou bien encore, un polymère acrylique ou méthacrylique ou un polyacrylamide. Un agent épaississant approprié est un copolymère acrylique et méthacrylique commercialisé sous la marque Carbopol 940 ou Carbopol 941.

Lorsqu'on veut préparer une préparation pulvérulente, on absorbe l'huile hyperoxygénée et le dérivé du rétinène sur un matériau inerte comme une silice colloidale, une argile ou la terre d'infusoires.

La poudre est ensuite diluée avec des excipients inertes comme le carbonate de calcium, le carbonate de Magnésium ou le Kaolin.

Les compositions pharmaceutiques selon l'invention sont destinées à être appliquées ou saupoudrées sur les lésions 2 à 3 fois par jour pendant une durée allant de 1 à 6 mois.

Les exemples suivants illustrent l'invention :

**EXEMPLE I**

| Crème à 1% d'acide rétinoïque et 1% d'huile hyperoxygénée de Maïs | |
|---|---|
| Acide rétinoique | 10 g |
| Huile hyperoxygénée de Maïs | 10 g |
| Huile de Paraffine | 150 ml |
| Stéarate de polyéthylène glycol | 1,5 g |

On dissout les principe actifs dans l'huile de parafine. On y incorpore progressivement sous forte agitation 85O ml d'une suspension aqueuse à 1% de silice. On obtient finalement une crème onctueuse, incolore, opaque que l'on parfume en y incorporant 5 ml de teinture de Benjoin.

**EXEMPLE II**

Démonstration clinique de l'efficacité thérapeutique des compositions selon l'invention.

GROUPE DE MALADES -1- :

|  | Taille moyenne de la lésion | Couleur | Infiltration | Consistance |
|---|---|---|---|---|
| Avant traitement | 5 cm x 2 cm | noire | + | Très dure |
| Après 15 jours | disparition | rose | – | Très molle |

Administration 2 fois/jour :

| Avant | 2 cm x 3 cm | noire | + | Très dure |
|---|---|---|---|---|
| Après | 1 cm x 2 cm | rosée | – | Très molle |

4

GROUPE DE MALADES –2–

| Avant traitement | 7 cm x 3 cm | noire | + | Très dure |
| --- | --- | --- | --- | --- |
| Après 15 jours | 5 cm x 1 cm | rouge | – | Très molle |

GROUPE DE MALADES –3–

| Avant traitement | 3 cm x 2 cm | noire | (–) | Très dure |
| --- | --- | --- | --- | --- |
| Après traitement | 2 cm x 1 cm | rouge | (–) | Très molle |

GROUPE DE MALADES –4–

| Avant traitement | 1 cm x 1 cm | noire/brune | (–) | Assez molle |
| --- | --- | --- | --- | --- |
| Après 15 jours | disparition | rosée | (–) | molle |

## Revendications

1. Compositions pharmaceutiques destinées principalement à la thérapeutique tumorale caractérisées en ce qu'elles renferment à titre de principes actifs, une huile hyperoxygénée et un dérivé oxygéné du rétinène, en association ou en mélange avec un véhicule ou excipient inerte, non irritant pharmaceutiquement acceptable.

2. Compositions pharmaceutiques selon la revendication 1 dans lesquelles l'huile hyperoxygénée est celle obtenue par aération ou oxygénation forcée d'une huile végétale ou animale.

3. Compositions pharmaceutiques selon la revendication 1° ou la revendication 2 dans lesquelles l'huile hyperoxygénée est celle obtenue par saturation en oxygène de l'huile de Maïs ou l'huile d'Arachide.

4. Compositions pharmaceutiques selon la revendication 1° das lesquelles le dérivé oxygéné du rétinène est choisi dans le groupe formé par le rétinol et ses esters, le rétinal, l'acide rétinoïque et les esters alcoyliques de l'acide rétinoïque.

5. Compositions pharmaceutiques selon la revendication 1° caractérisées en ce que le dérivé oxygéné du rétinène a la configuration cis ou la configuration trans.

6. Compositions pharmaceutiques selon la revendication 5° caractérisées en ce que le dérivé oxygéné du rétinène est l'acide rétinoïque ou la tretinoine.

7. Compositions pharmaceutiques selon l'une des revendications 1 à 6° dans lesquelles le rapport pondéral entre l'huile hyperoxygénée et le dérivé oxygéné du rétinène varie de 10 parties d'huile pour une partie de dérivé oxygéné du rétinène, à une partie d'huile hyper-oxygénée pour 5 parties de dérivé oxygéné du rétinène.

8. Compositions pharmaceutiques selon l'une des revendications 1 à 7° dans lesquelles l'excipient ou le véhicule est un de ceux qui conviennent pour l'application ou la pulvérisation par voie externe.

9. Une composition pharmaceutique selon l'une des revendications 1 à 7° caractérisée en ce qu'elle est présentée sous forme de lotion, de crème, de spray ou de poudre.

10. Une composition pharmaceutique selon l'une des revendications 1 à 9° caractérisée en ce qu'elle renferme de 1 à 2% de dérivé oxygéné du rétinène et de 0,5 à 5% d'huile hyperoxygénée par présentation unitaire.

11. Utilisation de l'acide rétinoïque et de l'huile hyperoxygénée obtenue par peroxygénation de l'huile de Maïs pour la réalisation d'un médicament destiné à traiter le syndrôme de Kaposi.

12. Procédé de préparation des compositions pharmaceutiques selon l'une des revendications 1 à 10° caractérisé en ce que l'on mélange ou on associe une huile hyperoxygénée et un dérivé oxygéné du rétinène à un ou plusieurs excipients ou véhicule inertes, non irritants pharmaceutiquement-acceptables selon les méthodes habituelles de la pharmacotechnie.

**Patentansprüche für folgende Vertragsstaaten : GR, ES**

1. Procédé de préparation des compositions pharmaceutiques destinées principalement à la thérapeutique anti-tumorale caractérisé en ce que l'on mélange ou on associe une huile hyperoxygénée et un dérivé oxygéné du rétinène à un ou plusieurs excipients ou véhicule inertes, non irritants pharmaceutiquement-acceptables selon les méthodes habituelles de la pharmacotechnie.

2. Un procédé de préparation des compositions pharmaceutiques selon la revendication 1° dans lequel l'excipient ou le véhicule est un de ceux qui conviennent pour l'application ou la pulvérisation par voie externe.

3. Un procédé de préparation des compositions pharmaceutiques selon l'une des revendications 1 et 2° caractérisé en ce que les compositions sont présentées sous forme de lotion, de crème, de spray ou de poudre.

4. Un procédé de préparation des compositions pharmaceutiques selon l'une des revendications 1 à 3° caractérisé en ce que les compositions renferment de 1 à 2% de dérivé oxygéné du rétinène et de O,5 à 5% d'huile hyperoxygénée par présentation unitaire.

6

Office européen
des brevets

RAPPORT DE RECHERCHE
EUROPEENNE

Numéro de la demande

**EP 90 40 2919**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| E | FR-A-2 645 747 (DIETLIN F. & FREDJ D.)<br>* le document en entier *<br>– – – | 1-12 | A 61 K 37/22<br>A 61 K 31/20 //<br>(A 61 K 37/22 |
| A | FR-M-2 330 (P. BARANGER)<br>* revendications 1-4 *<br>– – – | 1-12 | A 61 K<br>A 61 K 31:20<br>A 61 K 31:07 ) |
| A | EP-A-0 225 831 (S. DESJONQUERES)<br>* abrégé *<br>– – – | 1-12 | (A 61 K 31/20<br>A 61 K<br>A 61 K 31:07 ) |
| A | JOURNAL OF THE AMERICAN ACADEMY OF DERMATO-LOGY vol. 20, no. 6, juin 1989, FORT SAM HOUSTON, TEXAS pages 1123 - 1124; R. J. SHARPE: "Complete resolution of Kaposi's sarcoma with systemic etretinate therapy in a patient with mycosis fungoides"<br>* le document en entier *<br>– – – – – | 1-7 | |

**DOMAINES TECHNIQUES RECHERCHES (Int. Cl.5)**

A 61 K

Le présent rapport de recherche a été établi pour toutes les revendications

| Lieu de la recherche | Date d'achèvement de la recherche | Examinateur |
|---|---|---|
| La Haye | 13 juin 91 | LEHERTE C.F.M. |